# EUROPEAN PATENT APPLICATION

(11) **EP 1 321 164 A1**
(43) Date of publication of application: **25.06.2003**
(21) Application number: 01310839.4
(22) Date of filing: 21.12.2001
(51) Int. Cl.: A61N 1/02, A61B 5/0428, H01R 29/00

(54) **Indicator light**

(71) Applicant: Ultra Scientific Instruments Limited, Ashford, Kent TN23 6LN (GB)
(72) Inventor: Burnham, Hugh Alfred, Wye, Ashford, Kent TN25 5BD (GB); Jones, Roger Lomas, Greatstone, New Romney, Kent, TN28 8RJ (GB)
(74) Representative: Burke, Steven David

(57) **Abstract**

The present invention provides a mobile apparatus comprising a plurality of remote units (18) which can be placed in any of a number of arrangements, each remote unit having associated therewith an indicator light (25; 38) which is selectively activated in response to an activation signal from a control unit (12; 30) so that placement of the remote units according to any required arrangement can be achieved, and a plurality of cables (14; 32) extending between the control unit and respective remote units for carrying said activation signals and operation signals between the control unit and respective remote units.

The present invention also provides a muscle stimulation (10) apparatus comprising: a control unit (12) for controlling supply of electrical control signals to an arrangement of electrodes (18); and a plurality of cables (14) for supplying the electrical control signals to the electrodes along electrical conductors, each cable having associated therewith an indicator which is selectively activated in response to an indicator signal from the control unit (12).

The indicator or activation signals are optical signals and the cables (14) are provided with optical fibres for transmitting the indicator signals from the control unit (12) to the electrodes (18).

## Description

The present invention relates to apparatus comprising a control unit and a plurality of remote units controlled by the control unit. Particularly, but not exclusively, the invention relates to muscle stimulation apparatus.

Various apparatus are disclosed hereto which comprise a control unit, a plurality of remote units controlled by the control unit and a plurality of cables extending between the control unit and respective remote units. The remote units can be placed in any of a number of different arrangements, the position of each remote unit in the arrangement being related to the operation the unit has to perform. Since operation is controlled by the control unit it is essential that each remote unit is connected to the correct output from the control unit so that each remote unit is controlled correctly. Historically, this can be achieved by tracing the path of a cable from each output to each remote unit and placing that remote unit in the arrangement. However, with even only a small number of remote units this task becomes time consuming and is particularly so, when the cables are long and become tangled.

The above described problem is noticeable in medical apparatus in which the remote units may be placed on a human body (or animal body). Such medical apparatus include, without limitation, muscle stimulation apparatus, microcurrent apparatus, galvanic apparatus, interferential stimulator apparatus, ultrasound therapy apparatus, infrared therapy apparatus, electrocardiograph apparatus, electroencephlogram apparatus, polygraph apparatus and electroanalgisia apparatus. With such apparatus, the remote units are disposed in an arrangement on the patient and are operated to apply treatment to or to monitor a characteristic of the patient.

Of such medical apparatus, muscle stimulation apparatus will be described in more detail. Such apparatus are known which provide stimulation to selected muscles causing contraction and relaxation. Such stimulation replicates the effect of normal physical exercise and muscle stimulation apparatus may be used, for instance, in hospitals, beauty salons or in a domestic environment.

Prior art apparatus make use of wires or cables between a control unit and the electrodes placed on the body. Cables easily become tangled and since as many as a dozen leads may be used, the operator may become confused as to which lead does what.

As therapeutic techniques have developed, the benefits of being able to stimulate several groups of muscles on the body, during the same treatment session, have become apparent and this has led to complicated electrode placement diagrams, and a need for stimulating equipment which has a great many individually controlled outlets, contributing to further operator confusion.

In addition to medical apparatus, other types of non-medical apparatus which suffer from the above mentioned problem are, for example: seismic and ground resistance survey apparatus where a large number of probes or transducers are connected to measuring equipment; multichannel chart recording and oscilloscope apparatus where, for instance, a monitor monitors the output from sensors in a plurality of locations; computer apparatus where large number of cables may be used to interconnect a base unit to a plurality of peripheral units; audio and visual apparatus where, for instance, an amplifier or television, may be connected by cables to various different source components; and firework and explosive apparatus where a control unit is connected by cables to various detonators and explosives.

The problem mentioned above is made worse if the apparatus is mobile, or portable. Often, such apparatus must be completely disassembled and reassembled prior to and after moving. Cables can become tangled when moved leading to increased operator confusion.

The present invention is intended at least to mitigate the above problems.

Various aspects of the invention are set out in the accompanying claims.

According to a further aspect of the invention, a configurable apparatus comprises a plurality of remote units which can be placed in any of a number of arrangements, each remote unit having associated therewith an indicator which is selectively activated in response to an activation signal from a control unit to facilitate placement of the remote units according to a desired one of said arrangements, and a plurality of cables extending between the control unit and respective remote units for carrying said activation signals and operation signals between the control unit and respective remote units.

In order that the present invention may be well understood two embodiments thereof, which are given by way of example only, will now be described with reference to the accompanying drawings, in which:
Figure 1 is a simplified representation of a muscle stimulation apparatus in use on a subject;
Figure 2 is a schematic representation of a similar muscle stimulation apparatus; and
Figure 3 is a cross-sectional representation of a part of the muscle stimulation apparatus shown in Figure 2.

Although the embodiments described and illustrated hereinafter relate to muscle stimulation apparatus, it will be appreciated that the present invention has utility, without limitation, with the other types of medical and non-medical apparatus referred to above in which cabling is adopted and contributes to operator confusion. Since it will be readily appreciated from the illustrated embodiments how the present invention can be applied to other types of apparatus, further specific embodiments have not been described.

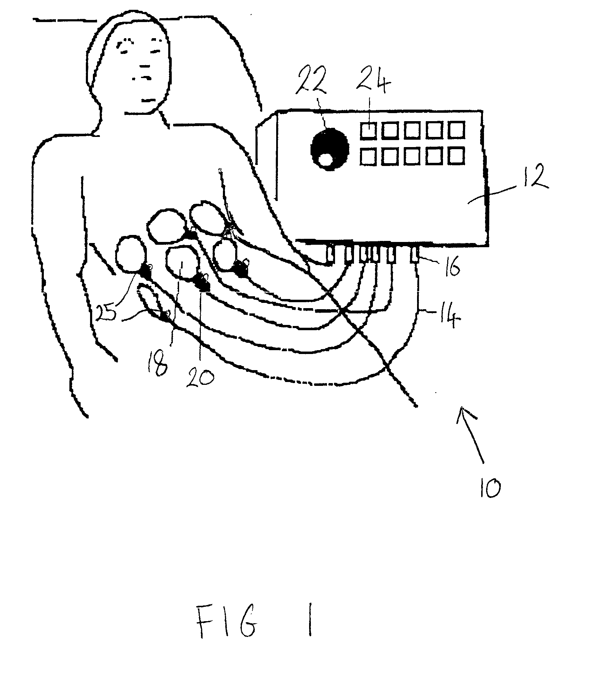

Referring to Figure 1, the muscle stimulation device 10 comprises a control unit 12. Cables 14 are connected to the control unit with plugs 16 and to an arrangement of electrodes 18 by connector means. The connector means comprise connectors 20 for connection to respective electrodes 18. The control unit 12 controls the supply of electrical stimulation signals along cables 14 to the connectors 20. The connectors 20 can be connected to respective electrodes 18 either before or after the electrodes are positioned on the body.

Muscles are generally overlaid by varying thickness of tissue and are of varying size. Therefore, it is a requirement that the electrical stimulation signal for each selected muscle is adjustable to achieve a comfortable and effective contraction. The electrical stimulation signal may be supplied as a pulsed voltage waveform and adjustment is achieved by altering the amplitude of the wave.

The control unit 12 comprises in this embodiment a rotary control 22 for adjusting the electrical stimulation signal supplied to the connectors 20 and thus, to the electrodes 18 when connected to the connectors 20. Push switches 24, typically ten, are provided for selecting individual connectors for adjustment. When a connector is selected, an indicator 25, associated with the cable connecting the selected connector 20 to the control unit, is activated to indicate to the operator which connector is being adjusted. In this embodiment, the indicators 25 are provided on the connectors 20 and take the form of a lens selectively illuminated by a light source provided in the control unit. Equally though, indicators could be provided on the cables themselves, for example, either at portions thereof proximate the electrodes, or along the entire length of the cable. The indicators may alternatively be provided on the electrodes 18, although this is not currently preferred because the electrodes may be disposable, for hygiene purposes.

Electrodes 18 are generally applied to the body in pairs, each electrode in a pair being positioned at selected points on the body. Push switches 24 may control the selection of pairs of connectors 20 for adjustment and indicate to the operator which two connectors 20 are being adjusted. When connected, the operator can then position the pair of electrodes appropriately.

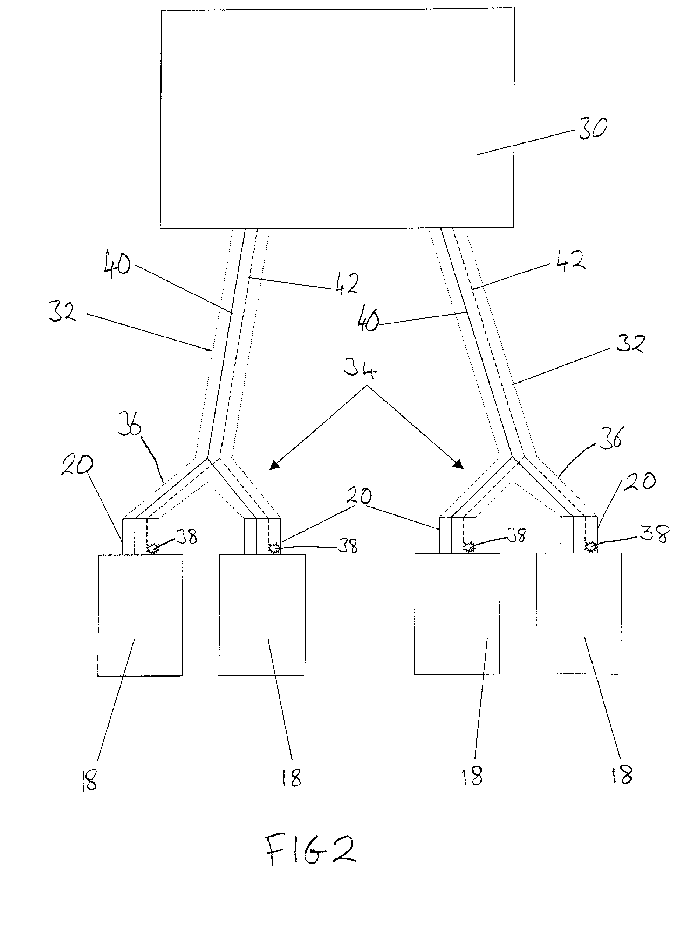

As will be described in more detail with reference to the muscle stimulator apparatus shown in Figure 2, the electrical stimulation signal is supplied to the electrode along electrical conductors which extend along cables 14. For safety reasons, isolation of a subject or patient is required from any interfering or hazardous current, including isolation from mains electricity, to protect the subject and operator from accidental electrical shocks. Isolation from the mains supply may be achieved by use of a transformer, in which the coils of the electrical circuit are separated from the coils of the mains supply, although such isolation may be achieved in other ways.

The indicators 25 may be light emitting diodes (LED's) or other electrically operated light emitting means for producing an optical signal. This would mean however that the electrical circuit for powering the light emitting means would also have to be isolated from the mains electricity.

Conveniently, the muscle stimulator apparatus makes use of light guides which, for example, may be provided by optical fibres extending along the cables 14 to provide indication of the selected connector or electrode. Respective light sources may be provided in the control unit which are selectively activated when respective push switches 24 are operated. The optical signal from the light source passes down an optical fibre extending along a cable 14 to a connector 20. At the end proximate the connector, the optical fibre may be provided with a multifaceted lens or prism (indicator 25) by which the light can be refracted or reflected in multiple directions to indicate to an operator which cable has been selected. An electrode can be connected to the selected connector and positioned on the body as appropriate. The advantage of using such an optical circuit is that the subject and operator are effectively isolated from the mains electricity supply. Preferably, the optical fibres are acrylic for purposes of electrical insulation and also, for cost of manufacture.

The muscle stimulation apparatus may be mobile in the sense that it can be moved from place to place to administer treatments. In some cases, the apparatus may be supported by a trolley or may be hand-held.

In use, an operator may consult an electrode placement diagram provided on a display or monitor of the control unit 12. The operator places electrodes on the body as directed either before or after connecting the electrodes to the connectors 20. The electrodes are then adjusted to the correct level as follows. The operator presses a push switch 24 which selects for adjustment one (or usually a pair) of electrodes 18. The indicator or indicators 25 associated with the selected electrodes indicates to the operator which electrodes are being adjusted. The operator carries out adjustment by rotating rotary control 22 according to the placement of the electrodes. Once the signal levels for the electrodes are adjusted, the apparatus is started and treatment begins. The indicators could be selectively activated during treatment to show which electrodes are currently in operation.

It is also possible for adjustment to be carried out prior to the connectors being connected to respective electrodes although this is not currently preferred.

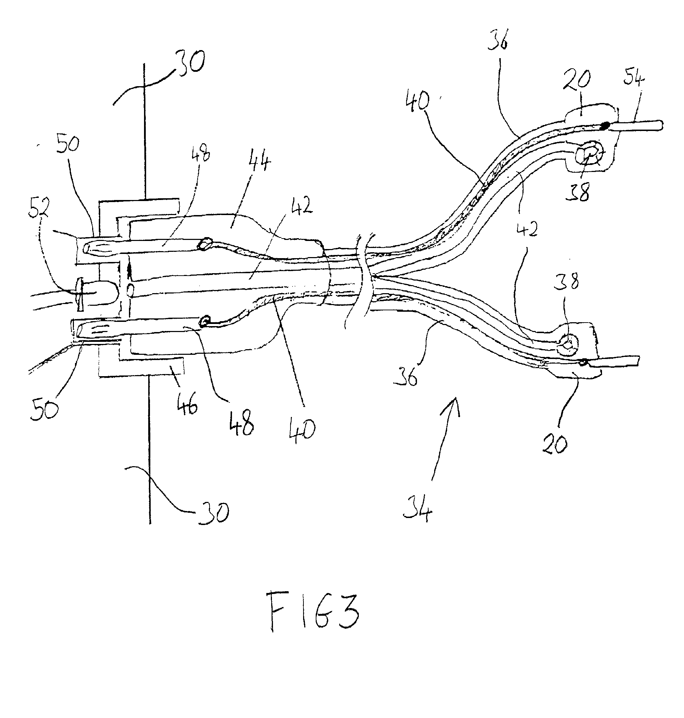

Figures 2 and 3 show a muscle stimulation apparatus, modified in that each cable has a main portion, for supplying control signals to a pair of electrodes, and connector means formed by two branches leading to respective individual connectors. With this arrangement, it is easier for an operator to identify pairs of electrode connectors as well as reducing the amount of cabling required.

Since the components of the apparatus shown in Figures 2 and 3 are similar like reference numerals will be used for like components where appropriate.

For simplicity in Figure 2, a control unit 30 is shown connected to only two cables 32. More cables may be used depending on how many electrodes are required for a particular treatment. Each cable 32 has connector means 34 for connecting the cable to a pair of electrodes 18. Each connector means 34 comprises two cable-branches 36 leading to two respective connectors 20. Each connector 20 is provided with an indicator 38 which may be in the form of a multi-faceted lens.

Each cable 32 is provided along its length with electrical conductors 40 and optical fibres 42. Both the electrical conductors and optical fibres branch into the cable-branches and lead to respective connectors 20.

Referring now to Figure 3 which shows the cable arrangement in more detail, each cable 32 is provided with a plug 44 for connection to a receptacle 46 in the control unit 30. Electrically conductive pins 48 extend from plug 44 for connection with electrical sockets 50. The pins 48 are in electrical connection with respective electrical conductors 40 so that when plug 44 is received in receptacle 46, electrical control signals can be transmitted from electrical sockets 50 to connectors 20.

Receptacle 46 comprises a light source 52 which may be an LED. Light source 52 is for emitting light to optical fibres 42 provided in plug 44. At least two optical fibres are required in the arrangement shown in Figure 3 so that one optical fibre can transmit light signals to each connector 20 in the pair. Preferably though, a bundle of optical fibres is provided, each fibre being of relatively small diameter. Such a bundle is relatively more flexible without causing damage to the fibres.

The optical fibre or bundle of optical fibres extends to each of the connectors 20 where a light transmitting means (indicator) is provided. This may be in the form of a multi-faceted lens to spread light in multiple directions. Alternatively, the light transmitting means may simply be the end or ends of the optical fibres although light transmitted in this way would not be so readily identified by an operator. It would also be possible to provide light transmitting means on any part of the connector means 34 whilst still enabling identification of a selected pair of connectors 20. It may also be desirable to provide the optical fibres with respective notches/dimples or to pigment the fibres to enable light to be transmitted from the fibres along their lengths.

The sheathing of the cables may be transparent and this is particularly advantageous if optical fibres are used which emit light along their lengths. It would also be possible to combine the light guide with the cable sheathing, for example, by providing the sheathing with an outer layer for guiding light along the cable.

Connectors 20 comprise electrically conductive electrode pins 54 for insertion into corresponding electrode sockets (not shown) provided in the electrodes 18.

The use of the apparatus described with reference to Figures 2 and 3 is similar to that described above with reference to Figure 1 and will be readily apparent from the above.

For hygiene reasons, particularly when either of the muscle stimulation apparatus as illustrated in Figure 1 to 3 are being used in a hospital or salon, the electrodes may be disposable. In this case, the apparatus may be supplied with or without electrodes, replacement electrodes being bought separately as and when required. It would not be desirable to provide disposable electrodes with the indicators, since it is preferred to keep the cost of manufacture of disposable items to a minimum. However, it would be possible to use reusable electrodes, in which case the indicators could be provided on the electrodes.

Instead of providing an indicator for each electrode, it would be possible to provide a single indicator for each pair of electrodes. This arrangement would be particularly suitable for the embodiment of Figures 2 and 3, where each cable 32 provided for driving a pair of electrodes could have associated therewith a single indicator instead of a pair thereof.

## Claims

1. A mobile apparatus comprising a plurality of remote units (18) which can be placed in any of a number of arrangements, each remote unit having associated therewith an indicator light (25; 38) which is selectively activated in response to an activation signal from a control unit (12; 30) so that placement of the remote units according to any required arrangement can be achieved, and a plurality of cables (14; 32) extending between the control unit and respective remote units for carrying said activation signals and operation signals between the control unit and respective remote units.

2. A mobile apparatus as claimed in claim 1, wherein said cables (14; 32) comprise light guides for guiding said activation signals from the control unit (12; 30) to the respective indicator lights (25; 38).

3. A mobile apparatus as claimed in claim 1 or 2, wherein the cables (14; 32) comprise connector means for connecting the cable to respective remote units, the respective indicator lights (25; 38) being provided on the connector means.

4. A mobile apparatus as claimed in claim 3, wherein each said indicator light (25; 38) is a lens or prism.

5. A mobile apparatus as claimed in either one of claims 3 or 4, wherein each cable (14; 32) has a bundle of light guides (42).

6. A mobile apparatus as claimed in any one of the preceding claims 2 to 5, wherein the light guides (42) emit light along their lengths.

7. A mobile apparatus as claimed in any one of the preceding claims, wherein said remote units are adapted to be placed on a human or animal body and are operable to supply stimulation to or receive information from such a body.

8. A muscle stimulation apparatus comprising: a control unit (12; 30) for controlling supply of electrical control signals to electrodes; and a plurality of cables (14; 32) for supplying the electrical control signals to the electrodes, each cable having associated therewith an indicator (25; 38) which is selectively activated in response to an indicator signal from the control unit (12; 30).

9. A muscle stimulation apparatus as claimed in claim 8, wherein each cable (14; 32) comprises connector means for connecting the cable to an electrode, the indicator (25; 38) being provided on the connector means.

10. A muscle stimulation apparatus as claimed in claim 9, wherein at least one said cable (14;32) is connected to a pair of electrodes (18) and has a connector means which comprises individual electrode connectors (20) each for connection to a respective electrode (18).

11. A muscle stimulation apparatus as claimed in claim 10, wherein the individual connectors (20) have provided thereon a said indicator (25; 38).

12. A muscle stimulation apparatus as claimed in claim 10, wherein a single indicator (25; 38) is provided for each pair of electrodes (18).

13. A muscle stimulation apparatus as claimed in any one of the preceding claims 8 to 12, wherein the indicator signals are optical signals transmitted to the indicators along light guides (42).

14. A muscle stimulation apparatus as claimed in claim 13, wherein each said indicator (25; 38) is a lens or prism.

15. A muscle stimulation apparatus as claimed in either one of claims 13 or 14, wherein each cable (14; 32) has a bundle of light guides (42).

16. A muscle stimulation apparatus as claimed in any one of the preceding claims 13 to 15, wherein the light guides (42) emit light along their lengths.

17. A muscle stimulation apparatus as claimed in any one of the preceding claims 13 to 16, wherein the light guides are optical fibres (42).

18. A medical apparatus comprising a plurality of remote units (18) which can be placed in any of a number of arrangements, each remote unit having associated therewith an indicator light (25; 38) which is selectively activated in response to an activation signal from a control unit (12; 30) so that placement of the remote units according to any required arrangement can be achieved, and a plurality of cables (14; 32) extending between the control unit and respective remote units for carrying said activation signals and operation signals between the control unit and respective remote units..
